# EUROPÄISCHE PATENTANMELDUNG

(11) **EP 2 111 893 A1**
(43) Veröffentlichungstag der Anmeldung: **28.10.2009**
(21) Anmeldenummer: 09156057.3
(22) Anmeldetag: 24.03.2009
(51) Int. Cl.: A61N 1/362

(54) **Ventrikulärer Herzstimulator**

(30) Priorität: 22.04.2008 DE 102008020123
(71) Anmelder: Biotronik CRM Patent AG, 6341 Baar (CH)
(72) Erfinder: Dörr, Thomas, 12437, Berlin (DE); Tscherch, Frank, 15741, Bestensee (DE); Tietze, Ulrich, 13156, Berlin (DE)
(74) Vertreter: Lindner-Vogt, Karin L.

(57) **Zusammenfassung**

Die Erfindung betrifft einen implantierbaren ventrikulären Herzstimulator (10), mit einem wenigstens zum Teil elektrisch leitenden Gehäuse (12), einem Elektrodenleitungsanschluss für den Anschluss einer ventrikulären Defibrillationselektrodenleitung (20) und einem Anschluss für eine Defibrillationselektrode (26). Erfindungsgemäß besitzt der Herzstimulator eine Fernfeldelektrokardiogrammerfassungseinheit (70), die einen ersten Eingang aufweist, der mit dem Anschluss für die ventrikuläre Defibrillationselektrode sowie einen zweiten Eingang, der mit dem elektrisch leitenden Gehäuse verbunden ist, und die ausgebildet ist, auf Basis der an den beiden Eingängen im Betrieb anliegenden elektrischen Potentiale ein Fernfeldelektrokardiogramm zu generieren. Der erfindungsgemäße Herzstimulator besitzt außerdem eine Fernfeldelektrokardiogrammauswerteeinheit (72), die mit der Fernfeldelektrokardiogrammerfassungseinheit verbunden und die ausgebildet ist, in einem von der Fernfeldelektrokardiogrammerfassungseinheit generierten Fernfeldelektrokardiogramm für atriale Depolarisationen charakteristische Signalmerkmale zu detektieren.

## Beschreibung

Die Erfindung betrifft einen implantierbaren ventrikulären Herzstimulator zur Stimulation des rechten oder des linken oder beider Ventrikel eines Herzens. Ein solcher Herzstimulator ist als Einkammer-Herzstimulator oder als biventrikulärer Herzstimulator bekannt und kann darüber hinaus auch die Funktion eines implantierbaren Cardioverters/Defibrillators (ICD) beinhalten.

Implantierbare Herzstimulatoren in Form von Herzschrittmachern oder Cardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- oder Defibrillationselektroden besitzen. Über eine Stimulationselektrode kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Um auf diese Weise eine stimulierte Kontraktion einer Herzkammer auszulösen, werden üblicher Weise Elektrodenleitungen mit relativ kleinflächigen Stimulationselektroden verwendet, da es zum Auslösen einer stimulierten Kontraktion einer Herzkammer ausreicht, wenn nur ein kleiner Teil des Myokards dieser Herzkammer anfänglich stimuliert wird. Eine derartige stimulierte Kontraktion einer Herzkammer wird im Rahmen dieser Beschreibung als stimuliertes Ereignis bezeichnet. Kommt es zu einer natürlichen Kontraktion der Herzkammer, wird dies im Rahmen dieser Beschreibung als Eigenaktion oder als intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

In Bezug auf die Stimulation einer Herzkammer, insbesondere des rechten oder des linken Ventrikels eines Herzens, ist weiter anzumerken, dass diese vorzugsweise atriumsynchron erfolgen, um möglichst gut die natürliche Kontraktionsfrequenz des Herzens abzubilden, bei der es zunächst zu einer Kontraktion des rechten Atriums und anschließend nach einer atrioventrikulären Überleitungszeit zu einer Kontraktion des rechten Ventrikels und gleichzeitig oder wenig später des linken Ventrikels kommt. Unter bestimmten Umständen unterbleibt bei einigen Patienten die natürliche Kontraktion des Ventrikels im Nachgang einer natürlichen Kontraktion des Atriums. In typischen Zweikammerschrittmachern wird daher die natürliche Kontraktion des Atriums als intrinsisches atriales Ereignis erfasst und nach einer vorgegebenen Überleitungszeit der rechte und/oder der linke Ventrikel stimuliert.

Das Erfassen solcher natürlichen (intrinischen) Ereignisse erfolgt durch Ableiten der elektrischen Potenziale des Myokards der jeweiligen Herzkammer mit Hilfe von Sensingelektroden, die Teil einer entsprechenden Elektrodenleitung sind. Dabei können die Sensingelektroden gleichzeitig die Stimulationselektroden sein und abwechselnd als Stimulations- und als Sensingelektroden verwendet werden. Typischer Weise ist für das Sensing ein Elektrodenpaar bestehend aus einer Spitzenelektrode (Tip-Elektrode) und einer Ringelektrode vorgesehen, von denen die Spitzenelektrode auch als Stimulationselektrode dient. Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat im jeweiligen Herzstimulator angeschlossen sind. Zusätzlich kann auch eine linksventrikuläre Elektrodenleitung vorgesehen sein, die typischer Weise über den Coronarsinus und eine von diesem abzweigenden, Lateralvene in die Nähe des linken Ventrikels ragt und dort eine kleinflächige Stimulations-und/oder Sensingelektrode aufweisen kann.

Die typischen Stimulationsmodi, die sich mit einem Herzstimulator verwirklichen lassen, können als bekannt vorausgesetzt werden (VVD, DDD usw.), so dass sie hier nicht weiter zu erläutern sind.

Ein implantierbarer Cardioverter/Defibrillator besitzt über die bereits beschriebenen Eigenschaften eines Herzschrittmachers hinaus weiterhin die Möglichkeit, einen stärkeren Stromimpuls an das Herz abzugeben, der nicht nur einen kleinen Teil des Myokards stimulieren (depolarisieren) soll, sondern einen möglichst großen Teil des Myokards depolarisieren und damit refraktär machen soll, um so eine für Fibrillationen typische kreisende Erregung des Myokards zu unterbrechen. Ein derartiger Impuls wird als Defibrillationsschock bezeichnet. Er wird typischer Weise über eine im Vergleich zur Stimulations- oder Sensingelektrode großflächige Defibrillationselektrode abgegeben.

Diese ist häufig in Form einer Schockwendel auf der Außenfläche der Elektrodenleitung in der jeweiligen Herzkammer realisiert. Beispielsweise kann eine ventrikuläre Elektrodenleitung neben einer Tip-Elektrode oder einer Ringelektrode für die Stimulation und das Sensing auch eine ventrikuläre Schockwendel und darüber hinaus auch eine nach Implantation in der Vene cava superior befindliche proximale Schockwendel besitzen.

Die Abgabe eines Defibrillationsschocks erfolgt in der Regel dann, wenn der Herzstimulator eine Fibrillation, d.h. eine unregelmäßige hochfrequente Eigenaktivität des Herzens, detektiert, die dazu führt, dass es zu keiner vollständigen Kontraktion der betroffenen Herzkammer kommt. Eine solche Fibrillation zählt zu den tachykarden Arrhythmien, zu denen neben Fibrillationen auch Tachykardien zählen. Im Unterschied zu einer Fibrillation folgt bei einer Tachykardie regelmäßig eine vollständige Kontraktion der betroffenen Herzkammer, jedoch mit einer Rate, die höher ist als physiologisch angemessen. Häufig können solche Tachykardien durch eine antitachykarde Stimulation behandelt werden und bedürfen nicht eines Defibrillationsschocks. Fibrillationen werden in der Regel mit einem Defibrillationsschock behandelt.

Bei tachykarden Tachykardien des Ventrikels unterscheidet man zwischen supraventrikulären Tachykardien (SVT) und ventrikulären Tachykardien (VT) im engeren Sinne. Letztere haben ihren Ursprung im Ventrikel selbst, während supraventrikuläre Tachykardien ihren Ursprung im Atrium haben. Für die nach Detektion einer Tachykardie eingeleitete Therapie ist die Art der ventrikulären Tachykardie (ventrikuläre Tachykardie im engeren Sinne (VT) oder supraventrikuläre Tachykardie (SVT)) von Bedeutung.

Das der Erfindung zugrundeliegende Problem besteht darin, dass eine atriumsynchrone Stimulation des Ventrikels oder aber auch die Diskriminierung (also Unterscheidung) zwischen supraventrikulären Tachykardien (SVT) und ventrikulären Tachykardien (VT) jeweils die Erfassung natürlicher atrialer Ereignisse voraussetzt. Dies geschieht nach dem Stand der Technik mit Hilfe atrialer Elektrodenleitungen, die entsprechende Sensingelektroden aufweisen. Anderen Herzstimulatoren, die nicht mit einer solchen atrialen Elektrodenleitung verbunden sind oder verbunden werden können, verschließen sich somit die entsprechenden Therapie- bzw. Detektionsmöglichkeiten.

Aufgabe der Erfindung ist es, Detektionsmöglichkeiten und Therapien, die eine Detektion atrialer Ereignisse voraussetzen, auch für solche Herzstimulatoren zu erschließen, die nicht mit einer atrialen Elektrodenleitung verbunden sind oder verbunden werden können, weil beispielsweise gar kein entsprechender Elektrodenleitungsanschluss bei dem Herzstimulator vorgesehen ist.

Erfindungsgemäß wird diese Aufgabe durch einen implantierbaren Herzstimulator gelöst, der ein Gehäuse besitzt, das wenigstens zum Teil eine elektrisch leitende Oberfläche besitzt sowie einen Defibrillationselektrodenleitungsanschluss für den Anschluss einer ventrikulären Defibrillationselektrodenleitung, wobei der Defibrillationselektrodenleitungsanschluss einen elektrischen Kontakt besitzt, der mit einem Gegenkontakt zu verbinden ist, welcher bei an den Herzstimulator angeschlossener Defibrillationselektrodenleitung mit deren Defibrillationselektrode elektrisch verbunden ist. Darüber hinaus weist der erfindungsgemäße Herzstimulator eine Fernfeldelektrokardiogramm-Erfassungseinheit auf, die einen ersten Eingang aufweist, der mit dem Anschluss für die ventrikuläre Defibrillationselektrode verbunden ist, sowie einen zweiten Eingang, der mit der elektrisch leitenden Oberfläche des Gehäuses des Herzstimulators verbunden ist. Die Fernfeldelektrokardiogramm-Erfassungseinheit ist ausgebildet, auf Basis der an den beiden Eingängen im Betrieb anliegenden elektrischen Potenziale ein Fernfeldelektrokardiogramm zu generieren. Außerdem besitzt der erfindungsgemäße Herzstimulator eine Fernfeldelektrokardiogramm-Auswerteeinheit, die mit der Fernfeldelektrokardiogramm-Erfassungseinheit verbunden und ausgebildet ist, in einem von der Fernfeldelektrokardiogramm-Erfassungseinheit generierten Fernfeldelektrokardiogramm für atriale Depolarisationen charakteristische Signalmerkmale zu detektieren. Atriale Depolarisationen kennzeichnen dabei atriale Eigenaktionen oder intrinsische (natürliche) atriale Ereignisse. Ein derartiger implantierbarer Herzstimulator kann auch ohne Kontakt zu einer Sensingelektrode im Atrium eines Herzens atriale Ereignisse detektieren und erschließt sich somit die Detektions- und Therapiemöglichkeiten, die auf der Detektion atrialer Ereignisse beruhen.

Der Herzstimulator besitzt vorzugsweise als ventrikulärer Herzstimulator eine ventrikuläre Stimulationseinheit, die mit einer linksventrikulären oder einer rechtsventrikulären Stimulationselektrode verbunden ist. Wie bereits eingangs erläutert, ist eine derartige Stimulationselektrode eine Elektrode, die eine wesentlich kleinere Oberfläche besitzt als die Defibrillationselektrode, die zur Aufnahme des Fernfeldelektrokardiogramms herangezogen wird. Eine solche ventrikuläre Stimulationselektrode ist beispielsweise eine Tip-Elektrode an einer ventrikulären Elektrodenleitung, kann aber auch eine Ringelektrode an einer ventrikulären Elektrodenleitung sein. Die ventrikuläre Stimulationseinheit ist darüber hinaus dazu ausgebildet, ventrikuläre Stimulationsimpulse zu generieren und abzugeben. Hierzu kann sie mit einer Stimulationssteuereinheit verbunden sein, die die ventrikuläre Stimulationseinheit ansteuert.

Ebenso kann der implantierbare Herzschrittmacher auch eine ventrikuläre Sensingeinheit besitzen, die über eine angeschlossene oder anzuschließende ventrikuläre Elektrodenleitung mit einer ventrikulären Sensingelektrode verbunden oder zu verbinden ist. Die ventrikuläre Sensingelektrode ist, ebenso wie die ventrikuläre Stimulationselektrode, eine im Vergleich zur Defibrillationselektrode kleinflächige Elektrode. Die ventrikuläre Sensingelektrode kann auch von einem Elektrodenpaar gebildet sein, beispielsweise einer ventrikulären Ring- und einer ventrikulären Tip-Elektrode, von denen beispielsweise die ventrikuläre Tip-Elektrode auch gleichzeitig Stimulationselektrode ist. Die ventrikuläre Sensingeinheit ist dazu ausgebildet, über die ventrikuläre Sensingelektrode elektrische Potenziale des Myokards aufzunehmen und auszuwerten und beispielsweise intrinsische ventrikuläre Ereignisse zu detektieren, indem die von der ventrikulären Sensingeinheit aufgenommenen elektrischen Potenziale jeweils mit einem Schwellwert verglichen werden. Überschreitet das elektrische Potenzial den Schwellwert, wird ein ventrikuläres Ereignis detektiert. Die Detektion kann auch auf die Auswertung eines gefilterten Potenzialverlaufs gestützt sein, bei der infolge Rauschens zufällig hohe Potenziale ausgefiltert sind. Die Detektion ventrikulärer Ereignisse mit Hilfe einer ventrikulären Sensingelektrode ist grundsätzlich bekannt.

Gemäß einer bevorzugten Ausführungsvariante des Herzstimulators besitzt dieser einen Defibrillationselektrodenleitungsanschluss, der für den Anschluss einer ventrikulären Defibrillationselektrodenleitung geeignet ist, die zwei Schockwendeln aufweist, nämlich eine proximale (beispielsweise zur Anordnung in der Vena cava superior vorgesehene) Schockwendel und eine distale Schockwendel. Dabei ist der Herzstimulator so ausgebildet, dass die beiden Schockwendeln unabhängig voneinander mit der Fernfeldelektrokardiogramm-Erfassungseinheit zu verbinden sind. Vorzugsweise ist diese dazu ausgebildet, von den beiden zur Verfügung stehenden Schockwendeln mit der proximalen Schockwendel verbunden zu sein, um das Fernfeldelektrokardiogramm aus der Potenzialdifferenz zwischen proximaler Schockwendel und elektrisch leitender Oberfläche des Herzstimulatorgehäuses zu bestimmen.

Gemäß einer alternativen Ausführungsvariante kann der Herzstimulator auch so ausgebildet sein, dass die Fernfeldelektrokardiogramm-Erfassungseinheit wahlweise mit einer proximalen oder einer distalen Schockwendel einer ventrikulären Defibrillationselektrodenleitung zu verbinden ist.

Die Fernfeldelektrokardiogramm-Auswerteeinheit ist vorzugsweise dazu ausgebildet, in dem Fernfeldelektrokardiogramm Senseereignisse durch Vergleich mit einem fest vorgegebenen oder einem variablen Schwellwert zu detektieren.

Um gezielt atriale Ereignisse als Senseereignisse in dem Fernfeldelektrokardiogramm zu detektieren, ist die Fernfeldelektrokardiogramm-Auswerteeinheit vorzugsweise ausgebildet, solche Senseereignisse in Form von Signalspitzen des Fernfeldelektrokardiogramms zu detektieren, die nicht einem intrinsischen atrialen Ereignis zuzuordnen sind und diese so detektierten Signalspitzen aus dem Fernfeldelektrokardiogramm zu entfernen. Die Fernfeldelektrokardiogramm-Auswerteeinheit ist dann weiterhin ausgebildet, das so gewonnene Fernfeldelektrokardiogramm zur Detektion atrialer Senseereignisse weiterzuverarbeiten. Diese atrialen Senseereignisse können dann wiederum durch Schwellwertvergleich aus dem um die übrigen Signalspitzen bereinigten Fernfeldelektrokardiogramm ermittelt werden.

Typische Signalspitzen im Fernfeldelektrokardiogramm, die nicht intrinsischen atrialen Ereignissen zuzuordnen sind, sind solche Signalspitzen, die auf ventrikuläre Ereignisse zurückgehen. Dementsprechend ist die Fernfeldelektrokardiogramm-Auswerteeinheit vorzugsweise mit der ventrikulären Sensingeinheit verbunden und ausgebildet, von der ventrikulären Sensingeinheit erfasste ventrikuläre Ereignisse entsprechende den Signalspitzen in dem Fernfeldelektrokardiogramm zuzuordnen, um die Signalspitzen zu bestimmen, die auf ventrikuläre Ereignisse zurückgehen. Die Fernfeldelektrokardiogramm-Auswerteeinheit ist dann dazu ausgebildet, solche Signalspitzen aus dem Fernfeldelektrokardiogramm zu entfernen.

Zusätzlich oder alternativ kann die Fernfeldelektrokardiogramm-Auswerteeinheit auch ausgebildet sein, ventrikulären Ereignissen zuzuordnende Signalabschnitte des Fernfeldelektrokardiogramms durch Signalformanalyse (Morphologieanalyse) zu detektieren, um die entsprechenden Signalabschnitte dann aus dem Fernfeldelektrokardiogramm zu entfernen. Besonders wirkungsvoll, wenn auch aufwändiger, ist es, wenn die Fernfeldelektrokardiogramm-Auswerteeinheit ausgebildet ist, die Zuordnung von Signalbestandteilen des Fernfeldelektrokardiogramms zu ventrikulären Ereignissen sowohl auf eine Morphologieanalyse als auch auf von der ventrikulären Sensingeinheit stammende Detektionssignale zu stützen.

Wie bereits erwähnt können die auf die zuvor beschriebene Weise ohne dezidierte atriale Sensingelektrode gewonnenen Signale zu atrialen Ereignissen für die Steuerung des Herzstimulators herangezogen werden. Besonders vorteilhaft ist es, wenn der Herzstimulator ausgebildet ist, durch die Detektion atrialer Senseereignisse gewonnene atriale Sensesignale so zu verarbeiten, dass unnötige ventrikuläre Stimulationen vermieden werden. Hierzu kann vorteilhaft sein, die in dem Fernfeldelektrokardiogramm detektierten Senseereignisse vor der Weiterverarbeitung zeitlich zu verzögern, um auf diese Weise eine bei herkömmlichen atriumsynchronen Herzschrittmachern bekannte Hysteresefunktion zu verwirklichen.

Ebenso ist es vorteilhaft, wenn atriale Sensesignale in einem linksventrikulären bzw. biventrikulären Herzstimulator zur Steuerung einer kardialen Resynchronisationstherapie (CRT) einschließlich AV-Zeitoptimierung genutzt werden. Gemäß weiterer bevorzugter Ausführungsformen ist der Herzstimulator ausgebildet eine kardiale Resynchronisationstherapie vorzugsweise einschließlich AV-Zeitoptimierung durchzuführen.

Wie ebenfalls bereits erwähnt ist die Detektion atrialer Ereignisse auch im Zusammenhang mit der Erkennung und Behandlung von Tachykardien und Fibrillationen nützlich. In einer bevorzugten Ausführungsvariante ist der Herzstimulator daher ein implantierbarer Cardioverter/Defibrillator (ICD), der ausgebildet ist, ventrikuläre Ereignisse zu detektieren und diese je nach Herzrate (bzw. Periode) ggf. einer Tachykardie oder einer Fibrillation zuzuordnen. Dies geschieht beispielsweise dadurch, dass das Intervall zwischen zwei aufeinander folgenden ventrikulären Senseereignissen oder der Kehrwert hiervon, nämlich die Herzrate, mit einem Schwellwert verglichen wird. Ist das ggf. über mehrere Zyklen gemittelte Intervall kleiner bzw. die ggf. gemittelte Herzrate größer als ein vorgegebener Schwellwert, detektiert der ICD eine Fibrillation. Liegt die Herzrate bzw. das Intervall zwischen aufeinander folgenden ventrikulären Ereignissen in einem Bereich, der noch nicht einer ventrikulären Fibrillation zuzuordnen ist, jedoch eine physiologisch unangemessen hohe Herzrate repräsentiert (dieser Bereich wird auch als VT-Zone bezeichnet), detektiert der Herzstimulator vorzugsweise eine ventrikuläre Tachykardie. Beispielsweise kann eine erste Grenzrate oder ein erstes Grenzintervall vorgesehen sein, welche bzw. welches als Vergleichswert für die Unterscheidung zwischen regulären Herzraten und Tachykardien dient, und eine zweite, höhere Grenzrate bzw. ein zweites, kürzeres Grenzintervall, welche bzw. welches als Vergleichswert für die Unterscheidung zwischen Tachykardien und Fibrillationen dient

Ein derartiger Herzstimulator ist vorzugsweise so ausgebildet, dass seine Fernfeldelektrokardiogramm-Auswerteeinheit atriale Ereignisse nur dann detektiert, wenn die zuvor geschilderte Analyse der ventrikulären Ereignisse ergibt, dass keine ventrikuläre Fibrillation, sondern allenfalls eine ventrikuläre Tachykardie vorliegt.

Weiterhin ist der Stimulator vorzugsweise dazu ausgebildet, mit Hilfe der Fernfeldelektrokardiogramm-Auswerteeinheit detektierte atriale Ereignisse eine Diskriminierung zwischen supraventrikulärer Tachykardie (SVT) und ventrikulärer Tachykardie (VT) vorzunehmen, indem das Auftreten ventrikulärer Ereignisse im zeitlichen Vergleich zum Auftreten atrialer Ereignisse ausgewertet wird. Insbesondere kann der Herzstimulator ausgebildet sein, nach Detektion einer ventrikulären Tachykardie eine antitachykarde Stimulation auszulösen. Beeinflusst diese antitachykarde Stimulation das zeitliche Auftreten atrialer Ereignisse, detektiert der Herzstimulator eine ventrikuläre Tachykardie (VT), andernfalls detektiert er eine supraventrikuläre Tachykardie (SVT), so wie dies in der parallel anhängigen deutschen Patentanmeldung (unser AZ 08.004) beschrieben ist.

Weiterhin ist ein Herzstimulator bevorzugt, der ausgebildet ist, eine atriumsynchrone Stimulation oder eine von der Detektion atrialer Ereignisse abhängige Elektrotherapie durchzuführen, sofern die Fernfeldelektrokardiogramm-Auswerteeinheit atriale Senseereignisse in dem Fernfeldelektrokardiogramm detektiert und andernfalls in einen Einkammerbetriebsmodus umschaltet, der nicht auf die Detektion atrialer Ereignisse angewiesen ist. Solche Einkammerbetriebsmodi für die ventrikuläre Stimulation (beispielsweise ein VVI-Modus) oder auch die Detektion und Diskriminierung ventrikulärer Tachykardien sind grundsätzlich bekannt. Ein Einkammerbetriebsmodus zur Diskriminierung ventrikulärer Tachykardien läuft darauf hinaus, dass der Herzstimulator ventrikuläre Tachykardien, die mit einer regelmäßigen Herzrate einhergehen, einer supraventrikulären Tachykardie (SVT) zuordnet und solche ventrikulären Tachykardien, die mit einer vergleichsweise variablen, schnellen Herzrate verbunden sind, einer eigentlichen ventrikulären Tachykardie (VT) zuordnet.

Weitere vorteilhafte Ausgestaltungen eines erfindungsgemäßen Herzstimulators ergeben sich aus hier nicht explizit erwähnten Kombinationen der verschiedenen vorteilhaften Merkmale eines solchen Herzschrittmachers, wie sie hier beschrieben sind.

Die Erfindung soll nun anhand eines Ausführungsbeispiels mit Bezug auf die Figuren näher erläutert werden. Von diesen zeigt:
- Fig. 1:: einen Herzstimulator in Form eines implantierbaren Einkammer-Kardioverter/Defibrillator in Verbindung mit daran angeschlossenen Elektrodenleitungen;
- Fig. 2:: ein schematisches Blockschaltbild des Herzstimulators aus Figur 1;
- Fig. 3:: einige Komponenten des Herzstimulators aus Figuren 1 und 2 in detaillierter Darstellung;
- Fig. 4:: ein mit Hilfe des implantierbaren Einkammer-Kardioverter/Defibrillators aus Figuren 1 und 2 gewonnenes Fernfeldelektrokardiogramm (unten) zusammen mit einem intraventrikulären Elektrokardiogramm (Mitte) und Markersignalen (oben);
- Fig. 5:: einen alternativen implantierbaren Einkammer-Kardioverter/Defibrillator in Verbindung mit daran angeschlossenen Elektrodenleitungen;
- Fig. 6:: einen implantierbaren biventrikulären Kardioverter/Defibrillator in Verbindung mit daran angeschlossenen Elektrodenleitungen;
- Fig. 7:: einen implantierbaren linksventrikulären Einkammer-Kardioverter/Defibrillator in Verbindung mit daran angeschlossenen Elektrodenleitungen;

- Fig. 8:: einige Komponenten des Herzstimulators aus Figur 5 in detaillierter Darstellung;
- Fig. 9:: ein mit Hilfe eines implantierbaren ventrikulären Kardioverter/Defibrillators gemäß Figuren 5 bis 8 gewonnenes Fernfeldelektrokardiogramm (unten) zusammen mit einem intraventrikulären Elektrokardiogramm (Mitte) und einem intraatrialen Elektrokardiogramm (oben);

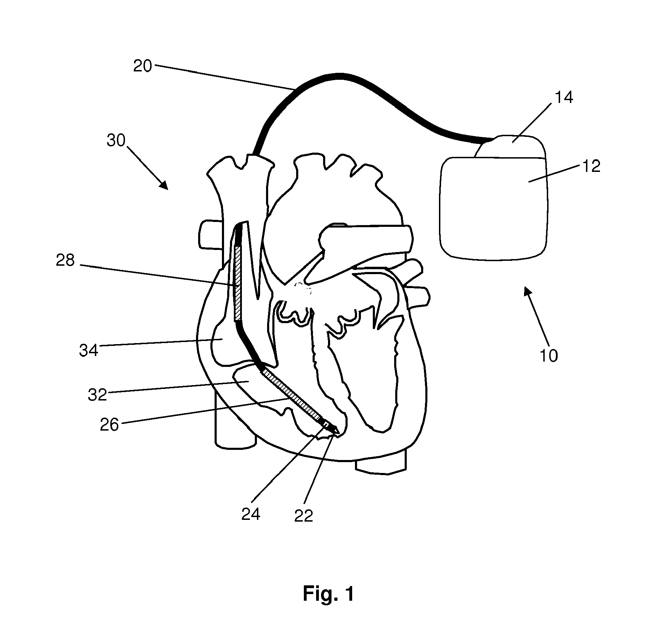

Fig. 1 zeigt einen ventrikulären Herzschrittmacher 10 mit einem Gehäuse 12 und einem Header 14. Das Gehäuse 12 ist hohl und besitzt wenigstens teilweise eine elektrisch leitende Oberfläche, typischer Weise besteht das Gehäuse 12 aus einem biokompatiblen Metall wie Titan. In dem Gehäuse 12 befinden sich eine Batterie und elektronische Komponenten des Herzstimulators 10. Der Header 14 enthält Anschlussbuchsen, beispielsweise für eine Elektrodenleitung 20.

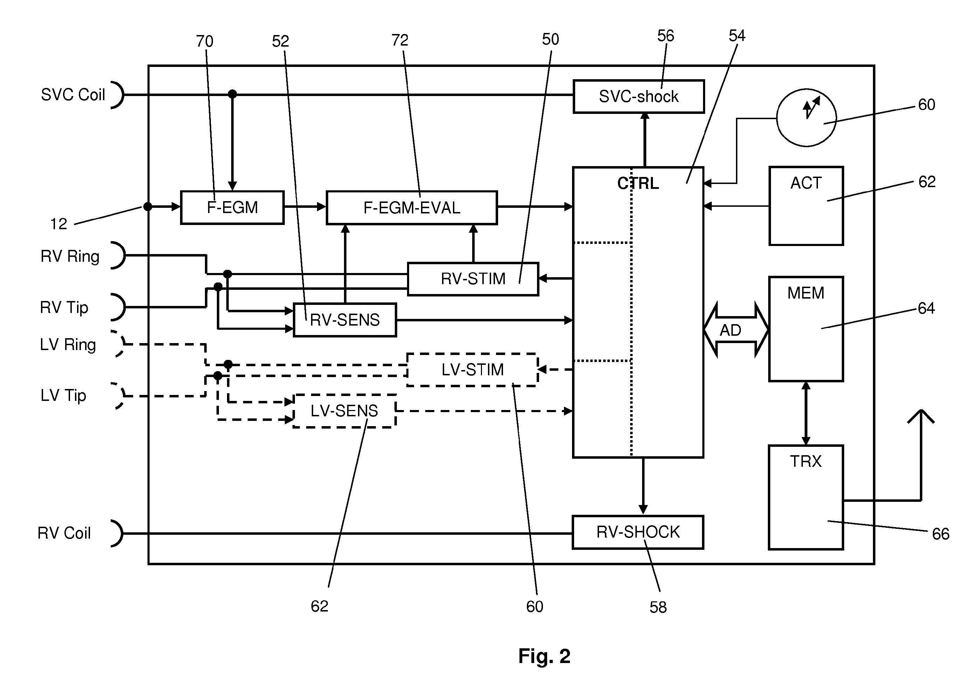

Die abgebildete Elektrodenleitung 20 ist eine ventrikuläre Elektrodenleitung mit einer ventrikulären Tip-Elektrode 22 und einer ventrikulären Ringelektrode 24. Von diesen dient die ventrikuläre Tip-Elektrode 22 als Stimulationselektrode. Die ventrikuläre Tip-Elektrode 22 und die ventrikuläre Ringelektrode 24 bilden zusammen ein Elektrodenpaar für das bipolare Sensing ventrikulärer Ereignisse. Hierzu sind die ventrikuläre Tip-Elektrode 22 und die ventrikuläre Ringelektrode 24 mit einer ventrikulären Stimulationseinheit und einer ventrikulären Sensingeinheit im Inneren des Gehäuses 12 des Herzstimulators 10 verbunden (Näheres ist mit Bezug auf Fig. 2 beschrieben). Außerdem besitzt die ventrikuläre Elektrodenleitung 20 eine ventrikuläre Schockwendel 26 als distale Schockwendel und eine proximale Schockwendel 28. Die ventrikuläre Schockwendel 26 ist dabei so auf der ventrikulären Elektrodenleitung 20 angeordnet, dass sie dann, wenn sie in ein Herz 30 eingeführt ist, im Ventrikel 32 des Herzens angeordnet ist. Die ventrikuläre Spitzenelektrode 22 befindet sich dann im Apex des Ventrikels 32 des Herzens 30. Die proximale Schockwendel 28 befindet sich bei implantierter ventrikulärer Elektrodenleitung 20 in der Vena cava superior des Herzens 30. Eine spezielle Elektrodenleitung zur Stimulation des rechten Atriums 34 des Herzens 30 ist nicht vorgesehen. Ebenso gibt es keine atriale Sensingelektrode. Der Herzstimulator 10 kommt ganz ohne atriale Elektrodenleitung aus und bietet dennoch die Funktionalität solcher Herzstimulatoren, die mit einer atrialen Elektrodenleitung verbunden sind. Dies wird anschließend näher erläutert.

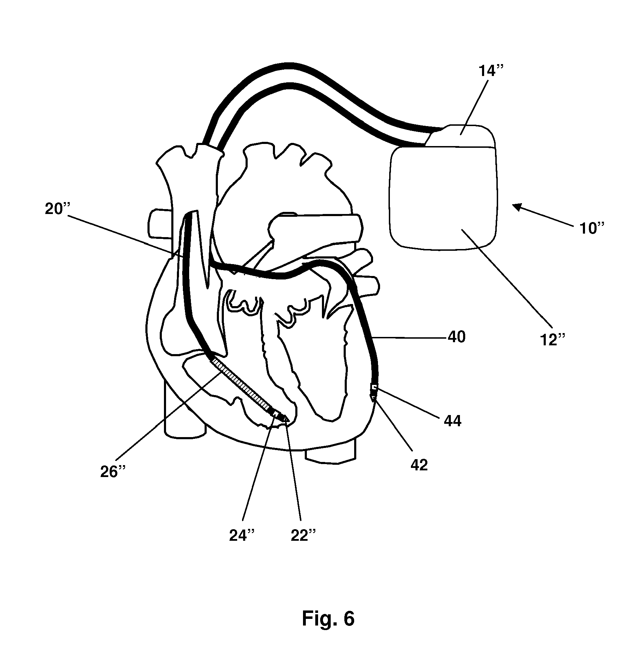

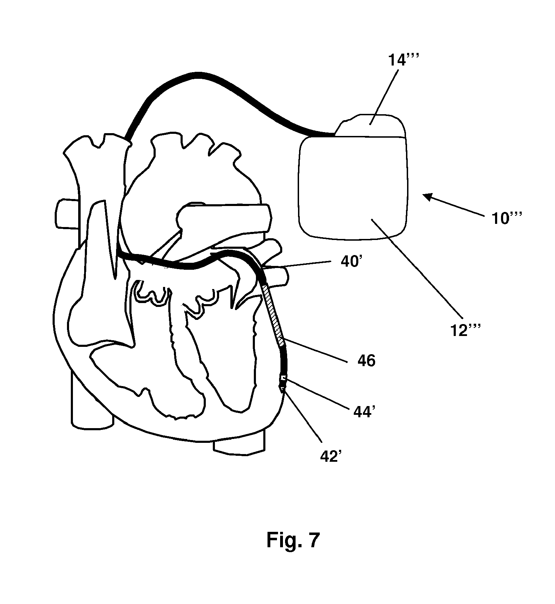

Fig. 2 zeigt in einem schematischen Blockschaltbild Komponenten des Herzstimulators 10, die im Inneren des Gehäuses 12 angeordnet sind. Dabei ist die Darstellung nicht notwendiger Weise abschließend. Insbesondere sind in Fig. 2 Komponenten gestrichelt dargestellt, die bei einem Herzstimulator 10 aus Fig. 1 nicht verwirklicht sind, sondern beispielsweise erst bei solchen Herzstimulatoren, wie sie beispielhaft in Figuren 6 oder 7 abgebildet sind.

Wie bereits angedeutet besitzt der Herzstimulator 10 in seinem Header 14 Anschlusskontakte für den Anschluss entsprechender Gegenkontakte eines Elektrodenleitungssteckers am proximalen Ende der Elektrodenleitung 20. Diese Kontakte dienen zur elektrischen Verbindung mit den Elektroden der Elektrodenleitung 20. So ist die proximale Schockwendel 38 mit dem Kontakt SVC Coil verbunden, die ventrikuläre (distale) Schockwendel 26 mit dem Anschluss RV Coil, die rechtsventrikuläre Tip-Elektrode 22 mit dem Anschluss RV Tip und die rechtsventrikuläre Ringelektrode mit dem Anschluss RV Ring. Über die Anschlüsse RV Tip und RV Ring sind die rechtsventrikuläre Tip-Elektrode 22 und die rechtsventrikuläre Ringelektrode 24 jeweils mit einer rechtsventrikulären Stimulationseinheit 50 und einer rechtsventrikulären Sensingeinheit 52 verbunden. Die rechtsventrikuläre Sensingeinheit 52 ist dabei ausgangsseitig mit einer Stimulationssteuereinheit 54 verbunden, die ihrerseits wiederum einen Ausgang besitzt, der mit der rechtsventrikulären Stimulationseinheit 50 verbunden ist. Die rechtsventrikuläre Stimulationseinheit 50 ist dazu ausgebildet, auf ein entsprechendes Steuersignal der Stimulationssteuereinheit 54 hin einen ventrikulären Stimulationsimpuls zu generieren und wenigstens über den Kontakt RV Tip abzugeben.

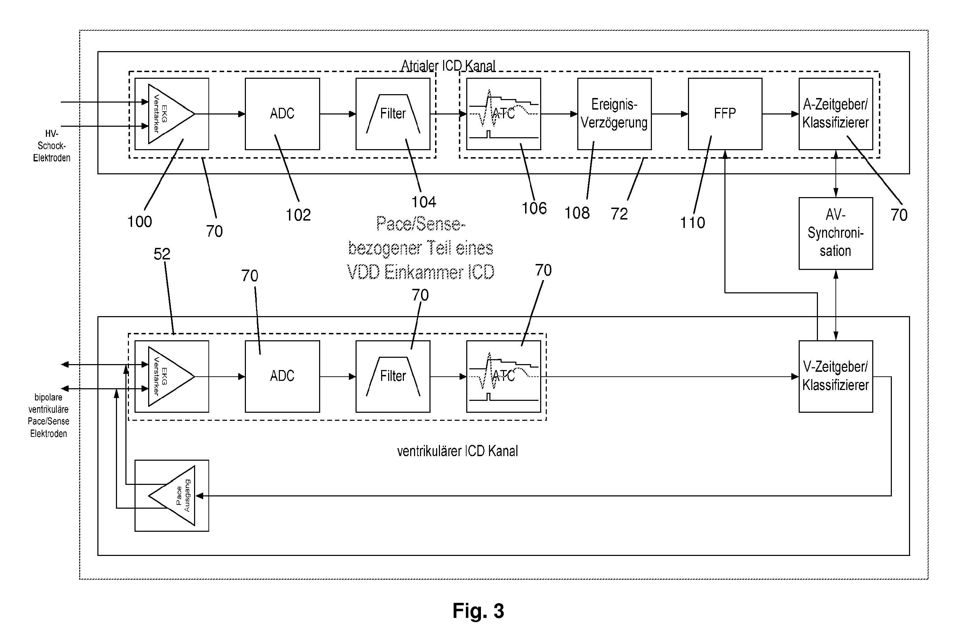

Die Funktionsweise der rechtsventrikulären Sensingeinheit 52 wird nachfolgend in Bezug auf Fig. 3 noch näher erläutert. Die rechtsventrikuläre Sensingeinheit ist grundsätzlich dazu ausgebildet, den Verlauf eines Signals auszuwerten, der sich aus der Differenz der an den Anschlüssen RV Tip und RV Ring anliegenden Potenziale ergibt. Dieser Signalverlauf enthält typischer Weise Signalspitzen, die im Falle ventrikulärer Depolarisationen auftreten. Ventrikuläre Depolarisationen gehen einer Kontraktion des ventrikulären Myokards voraus und kennzeichnen somit ventrikuläre Senseereignisse. Diese können aus dem Signalverlauf detektiert werden, indem die Potenziale mit einem Schwellwert verglichen werden. Dieser ist so eingestellt, dass die mit ventrikulären Depolarisationen einhergehenden Signalspitzen den Schwellwert überschreiten, so dass die ventrikuläre Sensingeinheit 52 ventrikuläre Senseereignisse durch Schwellwertvergleich bei Schwellwertüberschreitung detektieren kann.

Zur Erzeugung und Abgabe von Defibrillationsschocks sind außerdem Schockgeneratoren 56 und 58 vorgesehen, die über den Anschluss SVC Coil mit der proximalen Schockwendel 28 bzw. über den Anschluss RV Coil mit der distalen Schockwendel 26 verbunden sind. Die beiden Defibrillationsschock-Generatoren 56 und 58 sind dabei jeweils ebenfalls mit der Stimulationssteuereinheit 54 verbunden. Weitere Merkmale des Herzstimulators 10 sind ein Zeitgeber 60, der beispielsweise zur Intervallmessung und Herzratenbestimmung herangezogen wird, sowie ein Aktivitätssensor 62, der ausgebildet ist, eine physische Aktivität eines Patienten, beispielsweise durch Bewegungserfassung, zu erfassen, um es der Stimulationssteuereinheit 54 zu ermöglichen, eine Stimulationsrate an den physiologischen Bedarf eines Patienten anzupassen. Weiterhin besitzt der Herzstimulator 10 einen Speicher 64 zum Speichern von Steuerparametern sowie von physiologischen Parametern, die beispielsweise durch Auswerten der verschiedenen vom Herzstimulator 10 aufgenommenen Signale gewonnen werden.

Schließlich besitzt der Herzstimulator 10 auch eine Telemetrieeinheit 66, über die der Herzstimulator 10 gewonnene und gespeicherte physiologische Parameter an ein externes Gerät drahtlos übermitteln kann oder aber über die der Herzstimulator 10 Steuerparameter empfangen kann, die die Funktionsweise des Herzstimulators 10 steuern.

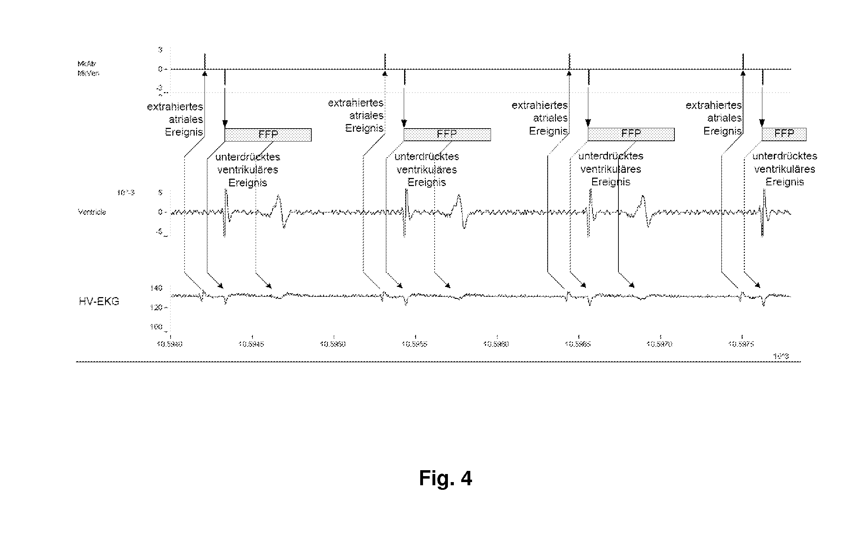

Im Sinne der Erfindung besitzt der Herzstimulator 10 außerdem eine Fernfeldelektrokardiogramm-Erfassungseinheit 70, die eingangsseitig einerseits mit dem elektrisch leitenden Gehäuse 12 des Herzstimulators 10 verbunden ist sowie andererseits mit dem Anschluss SVC Coil für die proximale Defibrillationselektrode bzw. Schockwendel 28. Aus der Potenzialdifferenz zwischen diesen beiden Eingängen ergibt sich ein Fernfeldelektrokardiogramm, das von der Fernfeldelektrokardiogramm-Erfassungseinheit 70 aufgenommen, verstärkt, analog-digital-gewandelt und gefiltert wird, wie dies mit Bezug auf Fig. 3 noch näher erläutert wird. Das so gewonnene und aufbereitete Fernfeldelektrokardiogramm liegt an einem Ausgang der Fernfeldelektrokardiogramm-Erfassungseinheit 70 an. Dieser Ausgang ist mit einem Eingang einer Fernfeldelektrokardiogramm-Auswerteeinheit 72 verbunden. Diese Fernfeldelektrokardiogramm-Auswerteeinheit 72 besitzt außerdem noch einen Eingang, der mit der rechtsventrikulären Sensingeinheit 52 sowie einen weiteren Eingang, der mit der rechtsventrikulären Stimulationseinheit 50 verbunden ist. Alternativ dazu kann auch ein einziger Eingang der Fernfeldelektrokardiogramm-Auswerteeinheit 72 vorgesehen sein, der mit der Stimulationssteuereinheit 54 verbunden ist. Diese weiteren Eingänge der Fernfeldelektrokardiogramm-Auswerteeinheit 72 dienen dazu, der Fernfeldelektrokardiogramm-Auswerteeinheit 72 Signale zuzuführen, die ventrikuläre Sensingereignisse bzw. ventrikuläre Stimulationsereignisse charakterisieren. Die Fernfeldelektrokardiogramm-Auswerteeinheit ist ausgebildet, das seitens der Fernfeldelektrokardiogramm-Erfassungseinheit 70 gebildete Fernfeldelektrokardiogramm unter Berücksichtigung ventrikulärer Sensingereignisse und Stimulationsereignisse charakterisierender Signale auszuwerten, um in dem Fernfeldelektrokardiogramm Signalmerkmale zu detektieren, die atriale (Sense-)Ereignisse charakterisieren. Auch dies ist mit Bezug auf die nachfolgenden Figuren 3 und 4 nachfolgend noch näher erläutert.

Von diesen Figuren zeigt Fig. 3 einige Komponenten der Fernfeldelektrokardiogramm-Erfassungseinheit 70 und der Fernfeldelektrokardiogramm-Auswerteeinheit 72 einerseits sowie der ventrikulären Sensingeinheit 52 andererseits in detaillierterer Darstellung. Zu beachten ist dabei, dass die Kombination aus Fernfeldelektrokardiogramm-Erfassungseinheit 70 und Fernfeldelektrokardiogramm-Auswerteeinheit 72 in Fig. 3 als atrialer ICD-Kanal bezeichnet ist und zwischen Fernfeldelektrokardiogramm-Erfassungseinheit und Fernfeldelektrokardiogramm-Auswerteeinheit nicht weiter differenziert.

Die Bestandteile des atrialen Kanals werden im Folgenden anhand der Funktionsweise erläutert. Das von der Defibrillationselektrode 38 abgeleitete Fernfeldelektrokardiogramm-Signal wird über einen Vorverstärker 100 einem Analog-Digital-Wandler (ADC) 102 zugeführt und anschließend durch ein digitales Bandpassfilter 104 digital bandpassgefiltert. Aus dem gefilterten Signal werden dann in einem Komparator 106 durch kontinuierlichen Vergleich mit einem algorithmisch nachgeführten Schwellwert Sense-Ereignisse gewonnen die durch ein Verzögerungsglied 108 zeitlich verzögert weiter verarbeitet werden. Es ist damit zu rechnen, dass so gewonnen Sense-Ereignisse nicht nur für die atriale Aktivität relevante Ereignisse anzeigen sondern auch solche die der ventrikulären Aktivität zuzuordnen sind. Daher werden in einer nachfolgenden Far Field Protection Einheit 110 eventuell aufgetretene dem Ventrikel zuzuordnenden Events für eine weitere Signalverarbeitung unterdrückt.

Deren Funktionsweise ist nachfolgend mit Bezug auf Figur 4 näher erläutert. Figur 4 zeigt beispielhaft einen Abschnitt eines Fernfeldelektrokardiogramms (untere Zeile) zusammen mit einem intraventrikulären Elektrokardiogramm (mittlere Zeile), welches mittels der ventrikulären Sensingelektroden 22 und 24 bipolar gewonnen wurde sowie mit atrialen und ventrikulären Markersignalen für die bewerteten Ereignisse im atrialen und ventrikulären Kanal. Das intraventrikuläre Elektrokardiogramm wurde im Ventrikel von der dort lokalisierten bipolaren Pace/Sense-Elektroden 22 und 24 mit Hilfe der unten in Figur 3 dargestellten Komponenten des ventrikulären Kanals (d.h. der ventrikulären Sensingeinheit) abgeleitet.

Mit Erfassen eines ventrikulären Ereignisses durch die ventrikuläre Sensingeinheit 52 generiert diese ein ventrikuläres Markersignal als ventrikuläres Sense-Signal und gibt dieses an die Far-Field-Protection-Einheit 110 aus. Diese startet daraufhin ausgehend von dem ventrikulären Ereignis ein Zeitfenster (FFP) in welchem solche Ereignisse, die eventuell in dem dem Fernfeldelektrokardiogramm zugeordneten atrialen Kanal auftreten können, unterdrückt werden, weil sie ursächlich dem Ventrikel zuzuschreiben sind, z.B. T-Wellen.

Im Ergebnis stehen so im atrialen Kanal nur die aus dem Fernfeldelektrokardiogramm extrahierten atrialen Ereignisse für eine synchronisierte ventrikuläre Stimulationssteuerung im VDD-Modus zur Verfügung.

Die so gewonnenen atrialen Ereignisse werden anschließend durch das Verzögerungsglied 108 mit einer positiven AV-Zeit-Hysterese beaufschlagt und der Stímulationssteuereinheit 54 zugeführt. Diese startet daraufhin in an sich bekannter Manier ein AV-Intervall (atrioventrikuläres Verzögerungsintervall), an dessen Ende ein ventrikulärer Stimulationsimpuls ausgelöst wird, falls die ventrikuläre Sensingeinheit nicht zuvor während des AV-Intervalls ein ventrikuläres Ereignis erfasst (Demand-Modus, ventrikuläre Stimulationsimpulse werden nur im Bedarfsfall ausgelöst). Durch die durch das Verzögerungsglied eingeführten AV-Zeit-Hysterese wird bewirkt, dass ventrikuläre Stimulationsimpulse nur ausgelöst werden, wenn auch bei mäßig verzögerter natürlicher AV-Überleitung keine ventrikulären Kontraktionen innerhalb des AV-Intervalls auftreten. Diese Funktion kann mittels Such- und Repetitivfunktionen erweitert werden und entspricht dann der derzeitigen "IRS plus" / "I-OPT" Funktion in bekannten Zweikammer-ICDs der Anmelderin.

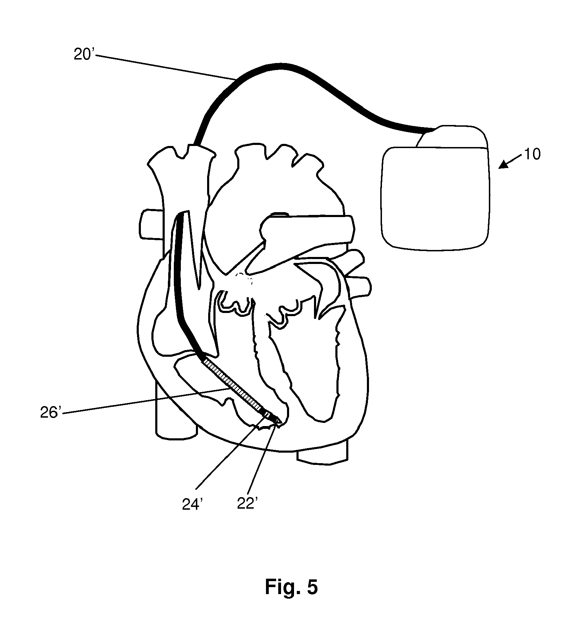

Die Figur 5 zeigt einen ventrikulären Herzstimulator 10' ähnlich dem aus Figur 1. Im Unterschied zu jenen trägt jedoch die rechtsventrikuläre Elektrodenleitung 20' nur eine Schockwendel 26' als Defibrillationselektrode im rechten Ventrikel. Dementsprechend ist die Fernfeldelektrogrammerfassungseinheit bei dem Herzstimulator 10' auch nicht mit einer Schockwendel in der Vena Cava Superior verbunden, sondern mit der rechtsventrikulären Schockwendel 26'. Im Übrigen gleichen sich die Herzstimulatoren aus Figur 1 und 5.

Wie Figur 6 zeigt, kann der erfindungsgemäße Herzstimulator auch als biventrikulärer Herzstimulator 10" ausgeführt sein, der neben einer rechtsventrikulären Elektrodenleitung 20" auch eine linksventrikuläre Elektrodenleitung 40 besitzt. Bei der Implantation wird die linksventrikuläre Elektrodenleitung 20" über den Coronar Sinus bis in eine von diesem abzweigende Lateralvene vorgeschoben, so dass sich ihr distales Ende im implantierten Zustand im Bereich des Myokards des linken Ventrikels eines Herzens befindet. Am distalen Ende der linksventrikulären Elektrodenleitung 40 ist eine linksventrikuläre Tipelektrode 42 als relativ kleinflächige Stimulations- und Sensingelektrode angebracht und in geringen Abstand davon eine linksventrikuläre Ringelektrode 44, die als Stimulations- und Sensingelektrode ebenfalls relativ kleinflächig ist.

Wie in Figur 2 bereits gestrichelt angedeutet, sind die beiden linksventrikulären Stimulations- und Sensingelektroden 42 und 44 jeweils mit einer linksventrikulären Stimulationseinheit 60 und einer linksventrikulären Sensingeinheit 62 verbunden.

Figur 7 zeigt, dass der erfindungsgemäße ventrikuläre Herzstimulator auch als rein linksventrikulärer Herzstimulator 10'" ausgebildet sein kann. Er ist im Betrieb ausschließlich mit einer linksventrikulären Elektrodenleitung 40' verbunden, die neben linksventrikulären Tip- und Ringelektroden 42' und 44' der bereits im Zusammenhang mit Figur 6 beschriebenen Art auch noch eine linksventrikuläre Schockwendel 46' als relativ großflächige Defibrillationselektrode. Letztere dient bei dem Herzstimulator 10"' aus Figur 7 zur Ableitung des Fernfeldelektrokardiogramms und ist mit der entsprechenden Fernfeldelektrokardiogrammerfassungseinheit verbunden.

Die Herzstimulatoren aus Figuren 6 und 7 erlauben eine kardiale Resynchronisationstherapie (CRT) ohne dass dazu eine atriale Elektrodenleitung erforderlich ist. Vielmehr erfolgt die atriale Sensesignalbestimmung wie zuvor beschrieben aus einem Fernfeldelektrokardiogramm. Die Herzstimulatoren 10" und 10"' aus Figuren 6 und 7 können die so gewonnene atriale Information, optional verbunden mit einer negativen AV-Zeit-Hysterese, nutzen, um eine AV-Zeit-optimierte Resynchronisationstherapie (CRT-Therapie) durch biventrikuläre oder linksventrikuläre Stimulation zu steuern. Der Header 14" des biventrikulären Herzstimulators 10" aus Figur 6 besitzt dazu zwei Anschlussbuchsen gemäß IS-1 Standard für den Anschluss der Stimulations- und Sensingelektroden und zwei Anschlussbuchsen gemäß DF-1 Standard für den Anschluss der Defibrillationselektroden.

Wie zuvor erwähnt, kann die Erfindung auch bei Herzstimulatoren Anwendung finden, die als implantierbare Cardioverter Defibrillatoren (ICD) ausgebildet sind. Bei diesen kann das wie zuvor beschriebene gewonnene Fernfeldelektrokardiogramm und die daraus abgeleitete Information über atriale Ereignisse in vorteilhafter Weise zur Unterscheidung von ventrikulären Tachykardien (VT) und supraventrikulären Tachykardien (SVT) herangezogen werden.

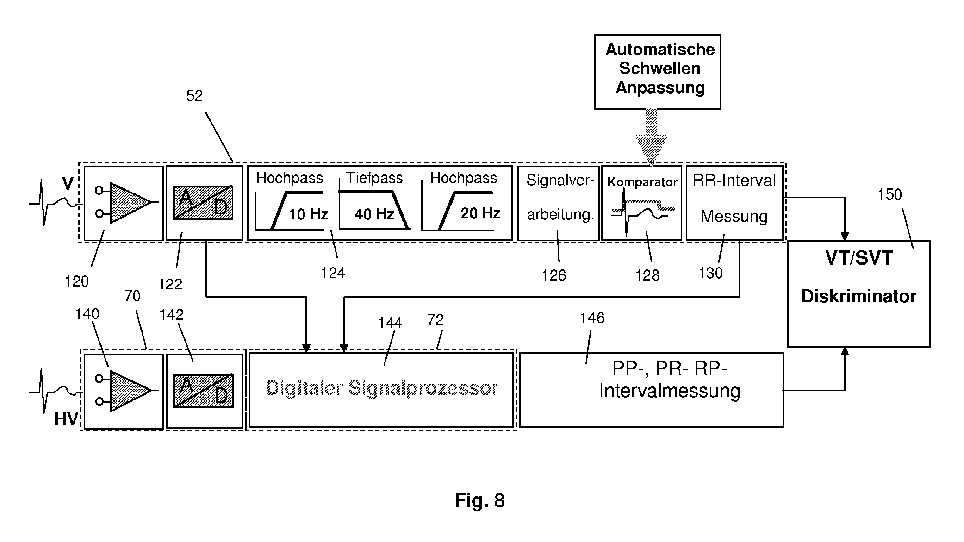

Figur 8 zeigt hierzu ein ausschnittsweises Blockschaltbild eines Einkammer-ICD mit erweiterter VT/SVT-Diskriminierung zur Darstellung der VT/SVT-Diskriminierung.

Über das Sensingelektrodenpaar 22 und 24 wird zunächst ein intraventrikuläres Elektrokardiogramm aufgenommen, das zunächst mittels eines Verstärkers 120 verstärkt, anschließend mittels eines Analog-Digital-Wandlers 122 digitalisiert und schließlich durch Filter 124 mehrstufig digital gefiltert wird. Anschließend führt eine Signalverarbeitungseinheit 126 eine Gleichrichtung und ggf. Invertierung des intraventrikulären Elektrokardiogramm-Signals, gefolgt von der Sense-Signal-Bestimmung in einer Komparatorstufe 128, die mittels automatisch mitgeführter Schwellenwerte R-Zacken als für ventrikuläre Depolarisationen typische Signalmerkmale in dem intraventrikulären Elektrokardiogramm bestimmt. Die R-Zacken werden dann einer RR-Intervall-Messeinheit 130 zur Bestimmung der RR-Intervalle, also der Intervalle zwischen jeweils aufeinander folgenden ventrikulären Ereignissen, weitergeleitet.

In einem zweiten, atrialen Sensingkanal wird das Fernfeldelektrokardiogramm, abgeleitet zwischen der proximalen Schockwendel und dem Gehäuse des Herzstimulators (HV), zunächst durch einen Verstärker 140 verstärkt und mittels eines Analog-Digital-Wandlers 142 digitalisiert. Anschließend wird in einem digitalen Signalprozessor 144 die P-Welle als für eine atriale Depolarisation charakteristisches Signalmerkmal unter Verwendung des intraventrikulären Elektrokardiogramms (Quelle: 20) aus dem Fernfeld-Elektrokardiogramm extrahiert und an eine nachgeschaltete PP-Intervall-Messeinheit 146 weitergeleitet. Die u. U. stromintensive Signalverarbeitung im digitalen Signalprozessor wird nur dann durchgeführt, wenn die gemessenen RR-Intervalle (d.h. die Intervalle zwischen aufeinander folgenden ventrikulären Ereignissen) innerhalb einer VT-Zone liegen.

Die von den beiden Intervall-Messeinheiten 130 und 146 bestimmten PP, PR, RP und RR
- Intervalle werden anschließend in einer VT/SVT-Bewertungseinheit 150 hinsichtlich der Ursache der Tachykardie bewertet. Vorzugsweise kommt hier der von Zweikammer-ICDs der Anmelderin bekannte SMART-Algorithmus unverändert zum Einsatz.

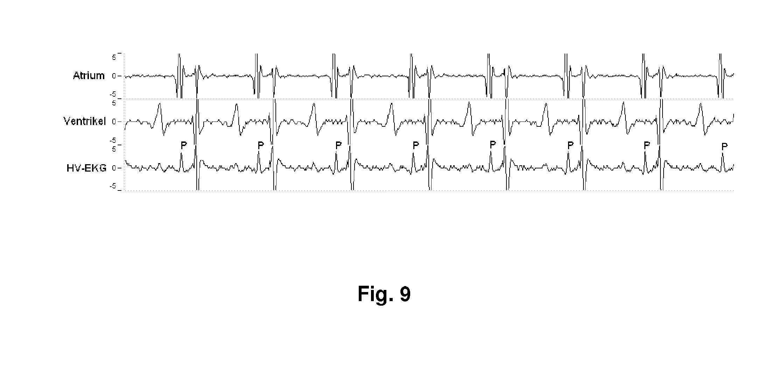

In Figur 9 ist unten ein Fernfeld-Elektrokardiogramm dargestellt, das zwischen der proximalen Schockwendel und dem Gehäuse des ICD abgeleitet wurde. Zusätzlich sind die Elektrokardiogramme aus dem rechten Atrium (oben) und rechtem Ventrikel (in der Mitte) dargestellt. In dem Fernfeld-Elektrokardiogramm sind die erkannten P-Wellen mit einem entsprechenden Marker (P) gekennzeichnet. Das abgebildete intraatriale Elektrokardiogramm kann von dem erfindungsgemäßen Herzstimulator nicht aufgenommen werden und ist nur zu Illustrationszwecken dargestellt.

## Patentansprüche

1. Implantierbarer ventrikulärer Herzstimulator (10), mit
- einem wenigstens zum Teil elektrisch leitenden Gehäuse (12), und
- einem Defibrillationselektrodenleitungsanschluss für den Anschluss einer ventrikulären Defibrillationselektrode (26; 28) mittels einer Defibrillationselektrodenleitung (20)
**gekennzeichnet durch**
- eine Fernfeldelektrokardiogrammerfassungseinheit (70), die einen ersten Eingang aufweist, der mit dem Anschluss für die ventrikuläre Defibrillationselektrode sowie einen zweiten Eingang, der mit dem elektrisch leitenden Gehäuse (12) verbunden ist, und
- die ausgebildet ist, auf Basis der an den beiden Eingängen im Betrieb anliegenden elektrischen Potentiale ein Fernfeldelektrokardiogramm zu generieren, sowie **durch**
- eine Fernfeldelektrokardiogrammauswerteeinheit (72), die mit der Fernfeldelektrokardiogrammerfassungseinheit (70) verbunden und
- die ausgebildet ist, in einem von der Fernfeldelektrokardiogrammerfassungseinheit (70) generierten Fernfeldelektrokardiogramm für atriale Depolarisationen charakteristische Signalmerkmale zu detektieren.

2. Implantierbarer Herzstimulator gemäß Anspruch 1, **gekennzeichnet durch** eine ventrikuläre Stimulationseinheit (50), die mit einer linksventrikulären oder einer rechtsventrikulären, gegenüber der Defibrillationselektrode (26, 28) relativ kleinflächigen Stimulationselektrode (22, 24) verbunden oder zu verbinden und ausgebildet ist, ventrikuläre Stimulationsimpulse zur Stimulation eines Ventrikels eines Herzens zu generieren und abzugeben.

3. Implantierbarer Herzstimulator gemäß Anspruch 1, **gekennzeichnet durch** eine ventrikuläre Sensingeinheit (52), die mit einer linksventrikulären oder einer rechtsventrikulären, gegenüber der Defibrillationselektrode (26: 28) relativ kleinflächigen Sensingelektrode (22, 24) verbunden oder zu verbinden und ausgebildet ist, elektrische Potentiale des ventrikulären Myokards aufzunehmen und zu verarbeiten.

4. Implantierbarer Herzstimulator gemäß Anspruch 1, **dadurch gekennzeichnet, dass** der Defibrillationselektrodenleitungsanschluss für den Anschluss einer ventrikulären Defibrillationselektrodenleitung (29) mit zwei Schockwendeln, nämlich einer proximalen Schockwendel (28) und einer distalen Schockwendel (26), derart ausgebildet ist, dass die Schockwendeln unabhängig voneinander mit der Fernfeldelektrokardiogrammerfassungseinheit (70) zu verbinden sind.

5. Implantierbarer Herzstimulator gemäß Anspruch 1, **dadurch gekennzeichnet, dass** der Defibrillationselektrodenleitungsanschluss für den Anschluss einer ventrikulären Defibrillationselektrodenleitung (20) mit zwei Schockwendeln, nämlich einer proximalen und einer distalen Schockwendel, ausgebildet ist und dass allein die proximale Schockwendel (28) mit der Fernfeldelektrokardiogrammerfassungseinheit (70) verbunden oder zu verbinden ist.

6. Implantierbarer Herzstimulator gemäß Anspruch 1**, dadurch gekennzeichnet, dass** die Fernfeldelektrokardiogrammauswerteeinheit (72) ausgebildet ist, in dem Fernfeldelektrokardiogramm Sense-Ereignisse durch Vergleich mit einem fest vorgegebenen oder variablen Schwellwert zu detektieren.

7. Implantierbarer Herzstimulator gemäß Anspruch 1, **dadurch gekennzeichnet, dass** die Fernfeldelektrokardiogrammauswerteeinheit (72) ausgebildet ist, in dem Fernfeldelektrokardiogramm Sense-Ereignisse (Signalspitzen) zu detektieren, die nicht einem intrinsischen atrialen Ereignis zuzuordnen sind und solche Signalspitzen aus dem Fernfeldelektrokardiogramm zu entfernen und anschließend das Fernfeldelektrokardiogramm zur Detektion atrialer Sense-Ereignisse weiterzuverarbeiten und/oder dass die Fernfeldelektrokardiogrammauswerteeinheit (72) ausgebildet ist, ein Fernfeldelektrokardiogramm einer Signalformanalyse zur Bestimmung von Signalspitzen zu unterziehen, die ventrikulären Ereignissen zuzuordnen sind.

8. Implantierbarer Herzstimulator gemäß Anspruch 3 und Anspruch 7, **dadurch gekennzeichnet, dass** die Fernfeldelektrokardiogrammauswerteeinheit (72) mit der ventrikulären Sensingeinheit (52) verbunden und ausgebildet ist, von der ventrikulären Sensingeinheit (52) erfasste Ereignisse als ventrikuläre Ereignisse Signalspitzen in dem Fernfeldelektrokardiogramm zuzuordnen und solche Signalspitzen aus dem Fernfeldelektrokardiogramm zu entfernen.

9. Implantierbarer Herzstimulator gemäß Anspruch 7, **dadurch gekennzeichnet, dass** die Fernfeldelektrokardiogrammauswerteeinheit (72) ausgebildet ist, in einem Fernfeldelektrokardiogramm ventrikulären Ereignissen zuzuordnende Signalspitzen auf Basis einer Signalformanalyse und unter Berücksichtigung von der ventrikulären Sensingeinheit (52) detektierten ventrikulären Ereignissen zu bestimmen und so bestimmte Signalspitzen anschließend aus dem Fernfeldelektrokardiogramm zu entfernen und das verbleibende Fernfeldelektrokardiogramm einem Schwellwertvergleich zur Detektion atrialer Ereignisse zu unterziehen.

10. Implantierbarer Herzstimulator nach einem der Ansprüche 1 bis 9, mit einer Stimulationssteuereinheit, die wenigstens mit der ventrikulären Stimulationseinheit verbunden ist, **dadurch gekennzeichnet, dass** die Fernfeldelektrokardiogrammauswerteeinheit (72) ausgebildet ist, atriale Sensesignale, die detektierte atriale Ereignisse repräsentieren, mit einer zeitlichen Verzögerung an die Stimulationssteuereinheit weiterzugeben.

11. Implantierbarer Herzstimulator gemäß Anspruch 10, **dadurch gekennzeichnet, dass** der Herzstimulator als Demand-Herzschrittmacher ausgebildet ist, dessen Stimulationssteuereinheit die Abgabe ventrikulärer Stimulationsimpulse inhibiert, falls die ventrikuläre Sensingeinheit innerhalb eines AV-Intervalls ventrikuläre Ereignisse erfasst, wobei die zeitliche Verzögerung einer atrioventrikulären Hysteresezeit entspricht und die Stimulationssteuereinheit ausgebildet ist, das AV-Intervall mit einem jeweils verzögerten atrialen Sensesignal zu starten.

12. Implantierbarer Herzstimulator nach einem der Ansprüche 1 bis 11, mit einem Anschluss für eine linksventrikuläre Elektrodenleitung und einer Stimulationssteuereinheit, die zur Abgabe einer kardialen Resynchronisationstherapie (CRT) ausgebildet ist.

13. Implantierbarer Herzstimulator nach einem der Ansprüche 1 bis 12, **dadurch gekennzeichnet, dass** der implantierbare Herzstimulator als implantierbarer Cardioverter / Defibrillator ausgebildet ist und eine VT/SVT-Bewertungseinheit aufweist, die ausgebildet ist, anhand von aufeinander folgenden erfassten atrialen Ereignissen und aufeinander folgenden ventrikulären Ereignissen eine Zuordnung einer ventrikulären Tachyarrhythmie zu einer supraventrikulären Tachykardie (SVT) oder einer ventrikulären Tachykardie (VT) durchzuführen.

14. Implantierbarer Herzstimulator gemäß Anspruch 13, **dadurch gekennzeichnet, dass** der Herzstimulator ausgebildet ist, ventrikuläre Ereignisse zu detektieren und diese in Abhängigkeit der Herzrate einer Tachykardie zuzuordnen wenn die Herzrate oberhalb einer ersten Grenzrate, aber unterhalb einer zweiten, höheren Grenzrate liegt, und einer Fibrillation zuzuordnen, wenn die Herzrate oberhalb der zweiten Grenzrate liegt, wobei der Herzstimulator weiterhin so ausgebildet ist, dass die Fernfeldelektrokardiogramm-Auswerteeinheit atriale Ereignisse nur dann detektiert, wenn die Herzrate unterhalb der zweiten Grenzrate liegt.

15. Implantierbarer Herzstimulator nach einem der Ansprüche 1 bis 14, **dadurch gekennzeichnet, dass** der implantierbare Herzstimulator ausgebildet ist, eine atriumsynchrone Stimulation oder eine von der Detektion atrialer Ereignisse abhängige Elektrotherapie durchzuführen, sofern die Fernfeldelektrokardiogramm-Auswerteeinheit atriale Senseereignisse in dem Fernfeldelektrokardiogramm detektiert und andernfalls in einen Einkammerbetriebsmodus umschaltet, der nicht auf die Detektion atrialer Ereignisse angewiesen ist.
